# EUROPEAN PATENT APPLICATION

(11) **EP 4 782 461 A1**
(43) Date of publication of application: **29.07.2026**
(21) Application number: 25382034.4
(22) Date of filing: 22.01.2025
(51) Int. Cl.: C07K 7/08, A61K 38/10, A61Q 11/00

(54) **A PEPTIDE OR PEPTIDE COCKTAIL AND ITS USE IN THE PREVENTION AND/OR TREATMENT OF AN ORAL INFLAMMATORY DISEASE**

(71) Applicant: DENTAID, S.L., 08290 Cerdanyola del Vallès (ES)
(72) Inventor: LEÓN BERRÍOS, Rubén, 08173 SANT CUGAT DEL VALLÈS (ES); BAÑÓ POLO, Manuel, 08029 BARCELONA (ES); ROMERO MARTÍNEZ, Rosa Maria, 08107 MARTORELLES (ES); TARRÉS MERCADER, Marina, 08950 ESPLUGUES DE LLOBREGAT (ES); ROPERO MORENO, Cristina, 08290 CERDANYOLA DEL VALLÈS (ES); GISPERT RIBAS, Juan, 50008 ZARAGOZA (ES); BLANC POCIELLO, Vanessa, 08019 BARCELONA (ES)
(74) Representative: Ponti & Partners, S.L.P

(57) **Abstract**

The present invention relates to a peptide or peptide cocktail and its use in the prevention and/or treatment of an oral inflammatory disease induced by oral pathogenic bacteria or any systemic disease which is negatively affected by said pathogenic bacteria.

## Description

### Field of the invention

The present invention relates to the field of peptides. In particular, the present invention relates to a peptide or peptide cocktail and its use in the prevention and/or treatment of an oral inflammatory disease induced by oral pathogenic bacteria or any systemic disease which is negatively affected by said pathogenic bacteria.

### Background

Periodontitis is one of the most prevalent diseases in the world and it is estimated that it may affect more than 1 billion people (1). In recent years, this disease has been defined as an inflammatory disease that affects the supporting tissues of the tooth and leads to the progressive and irreversible destruction of these tissues. The accumulation of biofilm around and under the subgingival margin promotes an immunoinflammatory response determined mainly by the presence of a specific bacterial community that interacts with the host's immune system. This inflammation normally begins with gingivitis (a reversible disease), which, if not treated promptly, can develop into periodontitis. The destruction of the periodontal ligament and alveolar bone is what ultimately causes tooth loss. The latter has a direct impact on people's quality of life, psychologically and in terms of nutrition, speech and socialisation. However, there is also substantial evidence linking periodontitis to the worsening of health conditions in systemic inflammatory diseases, such as diabetes, chronic obstructive pulmonary disease and certain cardiovascular diseases (2, 3).

Treating periodontitis is both expensive and complex, involving the thorough removal of calculus deposits and subgingival biofilm to reduce subgingival pockets and alleviate inflammation. However, this treatment is usually supplemented with antiseptics, as mechanical therapy alone is sometimes ineffective at controlling dysbiotic microbiota. For this reason, scaling and root planing are typically combined with the use of different antimicrobial substances, with chlorhexidine being considered the 'gold standard' antiseptic (4, 5). There is a general consensus regarding the use of antimicrobial therapy during periodontal treatment since the mechanical disruption of the biofilm allows for better penetration of the antiseptic and therefore better bacterial elimination (5).

The accumulation of a bacterial biofilm and the formation of calculus at and below the gingival margin are the most important risk factor for diseases that affect the supporting tissues of the tooth. For this reason, plaque control and removal play a fundamental role in the prevention and treatment of periodontal diseases (6). Several systematic reviews indicate that both mechanical control, through brushing, and chemical control of bacterial biofilm are effective in managing plaque build-up, which in turn helps reduce gingival inflammation (6, 7). Toothpastes, in general, regardless of their formulation, help control plaque. However, mouthwashes formulated with chlorhexidine, essential oils, and cetylpyridinium chloride prove to be more effective in preventing the formation of dental biofilm (6, 7).

For all these reasons, over the past 50 years, the oral hygiene products industry has developed a range of products (toothpastes and mouthwashes) formulated with different antiseptic molecules, primarily aimed at preventing the two main diseases caused by dental plaque: caries and periodontal disease. Some of the most commonly used molecules in toothpastes and mouthwashes are: stannous fluoride, amine fluoride, delmopinol, alexidine, chlorhexidine, cetylpyridinium chloride, essential oils, among others. All of these molecules, varying in effectiveness, provide generalised and non-specific antisepsis, acting on both pathogenic and commensal microbiota. Extensive literature highlights the clinical benefits and effectiveness of antiseptic mouthwashes in controlling oral pathogens. However, their impact on commensal microbiota, which is related to health, is insufficiently studied. Recently, next-generation sequencing techniques have shown that the use of chlorhexidine significantly reduces bacterial communities that convert dietary nitrates (NO₃⁻) to nitrite (NO₂⁻), and although its clinical relevance is not yet well established, these bacteria could contribute to cardiovascular health by promoting the production of nitric oxide (NO). This group of species, associated with health, is made up of bacteria from the genera *Veillonella, Actinomyces, Rothia, Granulicatella, Haemophillus* and *Neisseria*, and are found mainly in saliva and the dorsum of the tongue (8, 9). Nitric oxide is an essential signalling molecule that plays a key role in numerous physiological processes in mammals, such as cardiovascular homeostasis, inflammation, vascular tone, neurotransmission, and more. The oral microbiota's contribution to the enterosalivary circulation of NO₃⁻ and the production of NO₂⁻, leading to NO production, can play an important physiological role. For this reason, generalised antisepsis, which affects the organisation and functioning of commensal and/or health-related microbial communities can be highly undesirable for the development of products intended for the prevention and treatment of the most prevalent oral diseases (caries and periodontitis). The need to specifically control the load of pathobionts and/or pathogenic bacteria in the oral microbiota of most individuals drives the search for selective antisepsis that does not affect the commensal and/or health-related microbiota, promoting balance between the host tissues and the microbiota and helping to maintain oral cavity eubiosis.

Unlike conventional antimicrobials, which tend to lose their effectiveness when their molecular structure is altered, antimicrobial peptides (AMPs) exhibit great flexibility in their primary structure and can be modified or designed to target specific pathogens (10, 11). Most AMPs are small in size, 10 to 50 amino acids. This gives them structural flexibility to interact effectively with cell membranes, facilitating their diffusion and penetration through the membranes of bacteria and fungi. As they mainly attack cell membranes, they present a more complex target that is difficult to mutate without compromising cell viability (12). In addition, their rapid and multifaceted action reduces the likelihood that pathogens can develop resistance mechanisms (13). Their main mechanism of action is the disruption of cell membranes; however, they can also interfere with cellular processes or modify the immune response (14, 15).

Based on the above, the present invention has been developed to address the issues of generalised antisepsis resulting from the molecules currently used in oral hygiene products available on the market. The essence of the present invention lies in a strategy for achieving selective antisepsis, targeting oral pathogenic bacteria that trigger immune responses, promote inflammation of dental tissues and, potentially modulate or exacerbate other systemic inflammatory diseases (2, 3). Furthermore, most of these peptides have little or no antibacterial effect on commensal or health-related oral bacteria.

These novel peptides can be combined in various ways to target a range of species, enabling the development of prevention and treatment strategies for inflammatory dental diseases, including gingivitis, periodontitis, mucositis and peri-implantitis.

### Summary of the invention

In a first aspect, the present invention relates to a peptide comprising or consisting of the following amino acid structure:
K R W K W X₁ K Y X₂ H R Y F I X₃ H R R
wherein X₁ is selected from M, K, G, L, F, I and T;
wherein X₂ is selected from F, W, I, Y, L and V;
and wherein X₃ is selected from M, V, S, K, L, I and Q.

The present invention also relates to a peptide cocktail comprising or consisting of at least two or at least three of the peptides according to the first aspect of the invention.

In a second aspect, the present invention relates to the peptide or peptide cocktail according to the first aspect for use in the prevention and/or treatment of an oral inflammatory disease induced by oral pathogenic bacteria or any systemic disease which is negatively affected by said pathogenic bacteria.

### Bried description of the drawings

Figure 1.- Antimicrobial activity of peptide 434 on *F. nucleatum.* Tetracycline 20 µg/ml.
Figure 2.- Antimicrobial activity of peptide 353 on oral pathogenic bacteria. A: *F. nucleatum,* B: *F. alocis.* Tetracycline 20 µg/ml.
Figure 3.- Antimicrobial activity of peptide 355 on oral pathogenic bacteria. A: *F. nucleatum,* B: *F. alocis.* Tetracycline 20 µg/ml.
Figure 4.- Antimicrobial activity of peptide 449 on oral pathogenic bacteria. A: *F. nucleatum,* B: *F. alocis.* Tetracycline 20 µg/ml.
Figure 5.- Antimicrobial activity of peptide 413 on oral pathogenic bacteria. A: *F. nucleatum,* B: *P. gingivalis*, C: *P. intermedia*, D: *F. alocis.* Tetracycline 20 µg/ml.
Figure 6.- Antimicrobial activity of peptide 125 on oral pathogenic bacteria. A: *F. nucleatum,* B: *P. gingivalis*, C: *P. intermedia*, D: *F. alocis*, *E*: *P. endodontalis.* Tetracycline 20 µg/ml.

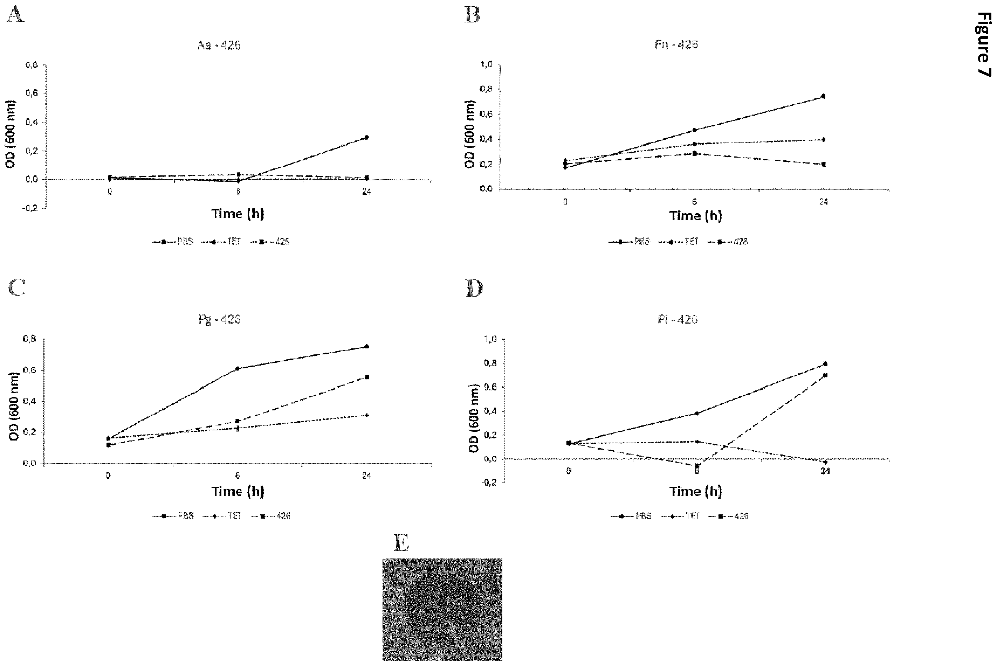

Figure 7.- Antimicrobial activity of peptide 426 on oral pathogenic bacteria. A: *A. actinomycetemcomitans,* B: *F. nucleatum* C: *P. gingivalis,* D: *P. intermedia,* D: *F. alocis.* Tetracycline 20 µg/ml.
Figure 8.- Antimicrobial activity of peptide 410 on oral pathogenic bacteria. A: *A. actinomycetemcomitans*, B: *F. nucleatum* C: *P. gingivalis*, D: *P. intermedia,* D: *F. alocis*, E: *P. endodontalis.* Tetracycline 20 µg/ml.
Figure 9.- Circular dichroism spectroscopy of the eight peptides recorded in the range of 190-260 nm. The ellipticity values are presented as a function of wavelength. A: peptide 434, B: peptide 353, C: peptide 355, D: peptide 449, E: peptide 413, F: peptide 125, G: peptide 426, H: peptide 410.
Figure 10. Effects of ionic strength on peptide stability. A: Stability was measured using intrinsic fluorescence in PBS at pH 7.4. B: Effect of weak ionic strength (0.1M NaCl). C: Effect of strong ionic strength (1M NaCl).
Figure 11.- Stability of the eight peptides at different pH and temperature conditions. A: pH, B: temperature
Figure 12.- Activity of the eight peptides on oral commensal bacteria. A: S. *mitis*, B: *S*. *oralis*, C: *S*. *salivarius*, D: *V. parvula*, E: *V. dispar*, F: *A. naeslundii*, G: *A. viscosus*, H: *N. subflava*, I: *R. mucilaginosa*, J: Diagram showing the distribution of the peptides across each plate.

### Detailed description

The present invention relates to a peptide comprising or, in another embodiment, consisting of the following amino acid structure:
K R W K W X₁ K Y X₂ H R Y F I X₃ H R R (SEQ ID NO: 1)
wherein X₁ is selected from M, K, G, L, F, I and T;
wherein X₂ is selected from F, W, I, Y, L and V;
and wherein X₃ is selected from M, V, S, K, L, I and Q.

The present invention also relates to a peptide cocktail comprising or, in another embodiment, consisting of at least two of the peptides according to SEQ ID NO: 1.

The present invention also relates to a peptide cocktail comprising or, in another embodiment, consisting of at least three of the peptides according to SEQ ID NO: 1.

In another preferred embodiment, a first peptide cocktail comprises or consists of the following peptides:
a1) K R W K W K K Y I H R Y F I V H R R (also named herein Peptide 353) (SEQ ID NO: 2)
a2) K R W K W G K Y W H R Y F I V H R R (also named herein Peptide 355) (SEQ ID NO: 3)
a3) K R W K W T K Y V H R Y F I Q H R R (also named herein Peptide 449) (SEQ ID NO: 4)

In another preferred embodiment, a second peptide cocktail comprises or consists of the following peptides:
b1) K R W K W M K Y F H R Y F I M H R R (also named herein Peptide 125) (SEQ ID NO: 5)
b2) K R W K W F K Y Y H R Y F I K H R R (also named herein Peptide 413) (SEQ ID NO: 6)
b3) K R W K W I K Y L H R Y F I L H R R (also named herein Peptide 426) (SEQ ID NO: 7)

In another preferred embodiment, a third peptide cocktail comprises or consists of the following peptides:
c1) K R W K W M K Y F H R Y F I M H R R (also named herein Peptide 125) (SEQ ID NO: 5)
c2) K R W K W L K Y I H R Y F I S H R R (also named herein Peptide 410) (SEQ ID NO: 8)
c3) K R W K W F K Y Y H R Y F I K H R R (also named herein Peptide 413) (SEQ ID NO: 6)

In another preferred embodiment, a fourth peptide cocktail comprises or consists of the following peptides:
d1) K R W K W M K Y F H R Y F I M H R R (also named herein Peptide 125) (SEQ ID NO: 5)
d2) K R W K W L K Y I H R Y F I S H R R (also named herein Peptide 410) (SEQ ID NO: 8)
d3) K R W K W I K Y Y H R Y F I I H R R (also named herein Peptide 434) (SEQ ID NO: 9)

In another preferred embodiment, the present invention relates to a composition comprising the peptide or peptide cocktail according to any of the previous embodiments, preferably said composition is a pharmaceutical composition. Said pharmaceutical composition further comprises a pharmaceutically acceptable vehicle, adjuvant, diluent or excipient, and optionally at least another active ingredient. Said pharmaceutically acceptable vehicle, adjuvant, diluent or excipient can be any of preserving agents, fillers, disintegrating agents, humidifying agents, emulsifying agents, suspending agents, solvents, dispersion media, coatings, isotonic and absorption delaying agents and the like. The use of such media and agents for pharmaceutical active substances is well known in the art. Except insofar as any conventional media or agent is incompatible with the peptide or peptide cocktail as defined in the present invention, its use in the pharmaceutical compositions is contemplated.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include also "and" unless the context clearly indicate otherwise.

It should be noted that the term "approximately" or "about" applied to the values used earlier and later in this document includes a margin of error of ± 5 %, such as, for example, ± 4 %, ± 3 %, ± 2 %, ± 1 %.

In a second aspect, the present invention relates to a peptide or peptide cocktail according to any of the previous embodiments alone or in combination or the composition comprising the peptide or peptide cocktail according to any of the previous embodiments alone or in combination for use in the prevention and/or treatment of an oral inflammatory disease induced by oral pathogenic bacteria or any systemic disease which is negatively affected by said pathogenic bacteria, such as, but not limited thereto, colorectal cancer, Alzheimer's disease.

In a preferred embodiment, said oral pathogenic bacteria are selected from *Porphyromonas gingivalies, Tannerella forsythia, Filifactor alocis, Fusobacterium nucleatum, Aggregatibacter actinomycetemcomitans, Prevotella intermedia, and Porphyromonas* endodontalis.

In another preferred embodiment, said oral inflammatory disease is selected from gingivitis, periodontitis, mucositis and peri-implantitis.

As used herein, the term "treat" or "treatment" or "treating" and their cognates refer to an amelioration/improvement of at least one discernible symptom of an oral inflammatory disease induced by oral pathogenic bacteria or any systemic disease which is negatively affected by said pathogenic bacteria. In another embodiment, "treat" or "treatment" or "treating" refer to an amelioration/improvement of at least one measurable physical parameter, not necessarily discernible by the patient. In another embodiment, "treat" or "treatment" or "treating" refer to inhibiting the progression of at least one discernible symptom of an oral inflammatory disease induced by oral pathogenic bacteria or any systemic disease which is negatively affected by said pathogenic bacteria. In another embodiment, "treat" or "treatment" or "treating" refer to slowing the progression or reversing the progression of at least one discernible symptom of an oral inflammatory disease induced by oral pathogenic bacteria or any systemic disease which is negatively affected by said pathogenic bacteria. As used herein, "prevent" or "prevention" or "preventing" and their cognates refer to delaying the onset or reducing the risk of acquiring or recurring at least one discernible symptom of an oral inflammatory disease induced by oral pathogenic bacteria or any systemic disease which is negatively affected by said pathogenic bacteria. The general terms "prevent" or "prevention" or "preventing" also include the prevention of recurrence in subjects that have had previous episodes of an oral inflammatory disease induced by oral pathogenic bacteria or any systemic disease which is negatively affected by said pathogenic bacteria.

As used herein, the term "preventing and/or treating" includes "preventing and treating" and "preventing or treating".

In another preferred embodiment, the peptide or peptide cocktail according to the present invention or the composition comprising thereof is used or administered in the form of a gel, toothpaste, lyophilized powders, chewing gum or chewable product.

The peptide or peptide cocktail or the compositions according to the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

The present disclosure also relates to a method of preventing and/or treating an oral inflammatory disease induced by oral pathogenic bacteria or any systemic disease which is negatively affected by said pathogenic bacteria, comprising the step of administering or using the peptide or peptide cocktail according to any of the embodiments disclosed herein alone or in combination or a composition, preferably a pharmaceutical composition, comprising said peptide or peptide cocktail according to any of the embodiments disclosed herein alone or in combination. In a preferred embodiment, said oral pathogenic bacteria are selected from *Porphyromonas gingivalies, Tannerella forsythia, Filifactor alocis, Fusobacterium nucleatum, Aggregatibacter actinomycetemcomitans, Prevotella intermedia and Porphyromonas* endodontalis. In another preferred embodiment, said oral inflammatory disease is selected from gingivitis, periodontitis, mucositis and peri-implantitis.

It is noted that any of the embodiments disclosed herein can be taken alone or combined with any other embodiment disclosed herein unless the context specifies otherwise. In other words, for example, a preferred option of a defined feature can be combined with a more or less preferred option of another feature.

The invention will be now illustrated with the following examples, which do not intend to limit its scope.

### EXAMPLES

### Library Design and Peptide Selection

As an example of the present invention, 8 peptides were selected from a library of 645. This library was designed using databases specialised in antimicrobial peptides, including: APD (Antimicrobial Peptide Database), CAMP (Collection of Antimicrobial Peptides), LAMP (Linking AMPs) and NDARO (NCBI National Database of Antibiotic Resistant Organisms). Filtering was performed using the following criteria: length (15-20 amino acids), net positive charge (+2 to +9), hydrophobicity (greater than 0.5), amphipathicity (0.2 to 0.8), and α-helix structure. Based on these parameters, tools such as PEP-FOLD and AlphaFold were used to predict the 3D structure and properties, including membrane insertion and pore-forming ability, while maintaining the α-helix structure. A polypeptide backbone was designed with aromatic and hydrophobic amino acids as the core, while various positions within the sequence were randomised.

For the present work, the peptides were chemically synthesised in the company Macrogen Co. Ltda, with a guaranteed purity of over 90%.

### Evaluation of Antibacterial Activity

An initial screening was performed on *Escherichia coli* DH5α (Thermo Fisher Scientific). Briefly, the bacteria were grown in Luria-Bertani medium until logarithmic phase and diluted to 10⁶ cfu/mL. The cells were placed in 96-well plates and the peptides were added to a final concentration of 50 µM; it was incubated at 37°C for 18-24 hours in a Varioskan Lux (Thermo Fisher); the optical density (600 nm) was measured every 30 minutes. The positive and negative controls were, medium plus bacteria, medium plus tetracycline and medium plus bacteria plus Phosphate Buffered Saline (PBS), respectively.

### Oral Bacterial Strains

Periodontopathogens: *Fusobacterium nucleatum* (DSM 20482), *Phorphyromonas gingivalis* (ATCC 33277), *Aggregatibacter actinomycetemcomitans* (DSMZ 8324), *Prevotella intermedia* (ATCC 25611), *Tannerella forsythia* (ATCC 43037), *Filifactor alocis* (ATCC *35896), Phorphyromonas endodontalis* (ATCC 35406).

Commensal bacteria: *Streptococcus oralis* (CECT 907 T), *Streptococcus sanguinis* (NCTC *10904), Actinomyces viscosus* (ATCC 15987), *Actinomyces naeslundii* (DSM 17233), *Streptococcus mitis* (DSM 12643), *Veillonella díspar* (DSM 20735), *Veillonella parvula* (ATCC 10790), *Rothia mucilaginosa* (clinical isolate), *Neisseria subflava* DSM 17610.

### Effect of Peptides on Oral Bacteria

The antibacterial activity of peptides on the bacteria *F. nucleatum, P. gingivalis, A. actinomycetemcomitans, P. intermedia, V. párvula, V. dispar, A. naeslundii, A. viscosus, S. sanguinis, S*. *oralis* and *S. mitis,* was performed similarly to that described previously for *E*. *coli.* The final concentration of the peptides was 50 µM. In this case, all species were grown anaerobically, in a mucin-free medium described in Blanc et al, 2013. The optical density was monitored at 6 and 24 hours in Varioskan Lux (Thermo Fisher). Tetracycline was used as an inhibition control.

For the species *F. alocis, T. forsythia* and *P*. *endodontalis*, antimicrobial activity was determined by drop test. Briefly, bacteria previously grown in liquid medium were plated as a lawn on non-selective medium [blood agar No. 2 (Oxoid) with 5% defibrinated horse blood, hemin (5 mg/L) and menadione (1 mg/L)], and 20 µl of each peptide were then added to the surface of the previously inoculated bacteria. All assays were performed in triplicate.

### Structural Characterisation of the Peptides

Structural characterisation was performed using circular dichroism (CD) spectroscopy to determine whether the peptides adopted an α-helix conformation or secondary structure elements.

### Peptide Stability

The stability of the peptides to protease K (1 mg/ml) was studied at a concentration of 50 µM and physiological pH (pH 7.4 in PBS). The preparations were incubated at room temperature and at 37 °C for 30 minutes. Proteinase K was inactivated at 4 °C. The preparations were then incubated at 37 °C and fluorescence was measured at 280 nm at 6, 24, 48 and 72 hours.

Stability was analysed across various pH levels: acidic (pH 4-6), neutral (pH 7-8), and basic (pH 9-11). The different solutions were incubated at 37 °C, and fluorescence was measured at 280 nm at 6, 24, 48 and 72 hours.

Finally, the thermal stability of the peptides was determined. Peptide solutions at pH 7.0 were incubated at 4, 25, 37 and 50 degrees Celsius. Fluorescence at 280 nm was measured at 6, 24, 48 and 72 hours. All assays were performed in triplicate, and peptides were left at a final concentration of 50 µM.

After the stability assays, the activity of the peptides on *P*. *gingivalis* and *F. nucleatum* was tested, as described above.

### Results

### Peptide Selection

Briefly, the 645 peptides comprising this library were initially screened against *E. coli.* Subsequently, their antimicrobial activity against 7 oral pathogenic bacteria was determined and, finally, their effect on 9 species of oral commensal bacteria was determined. The peptides that demonstrated the highest activity against pathogenic bacteria and the lowest activity against commensal bacteria were selected. Finally, these peptides were subjected to a final cytotoxicity screening using the standard protocol "CellTiter 96^{®} AQueous One Solution Cell Proliferation Assay" from Promega; the cells used were primary gingival fibroblasts; Normal, Human, Adult (PCS-201-018^{™} ATCC). Following this selection process, a total of 8 peptides showed the best results.

### Antimicrobial activity of the selected peptides

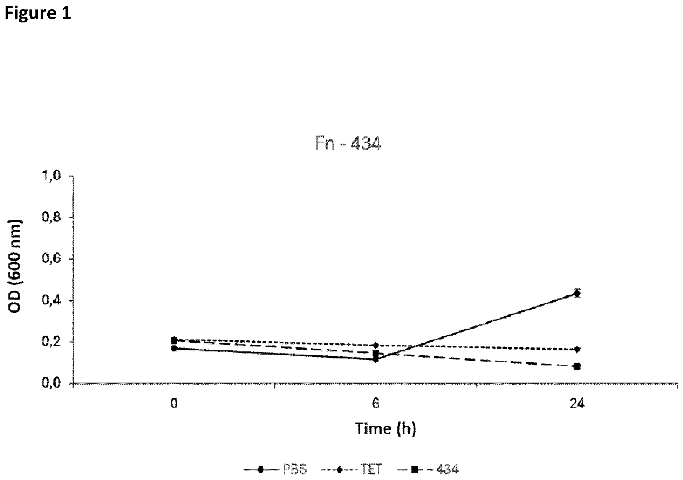

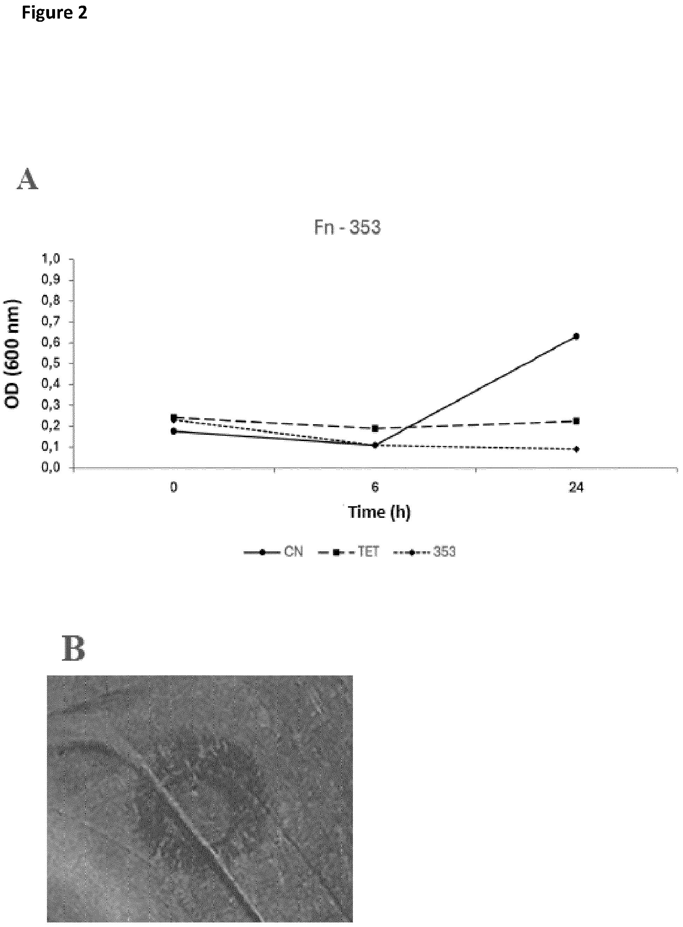

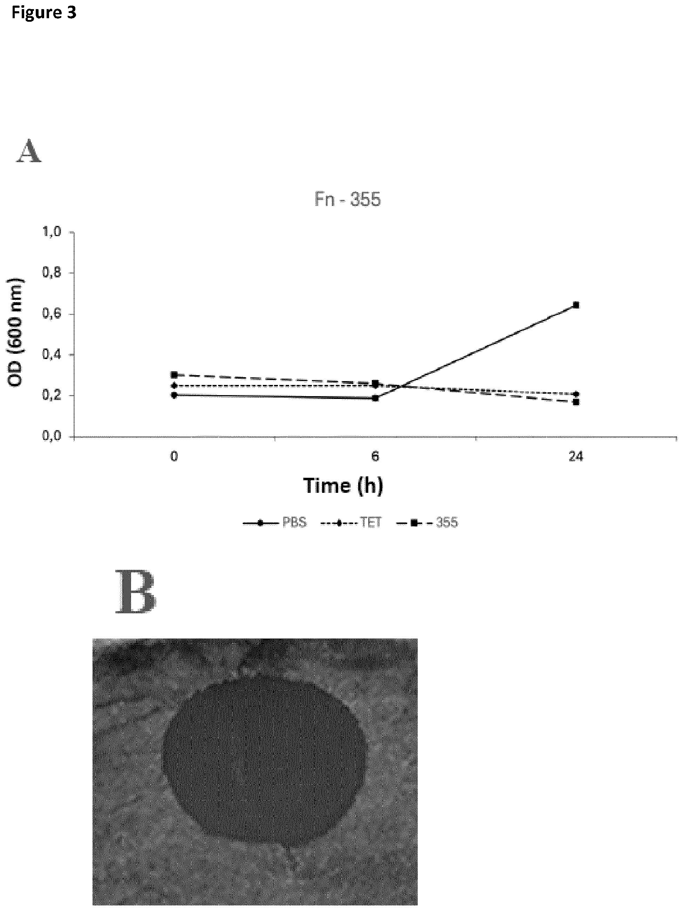

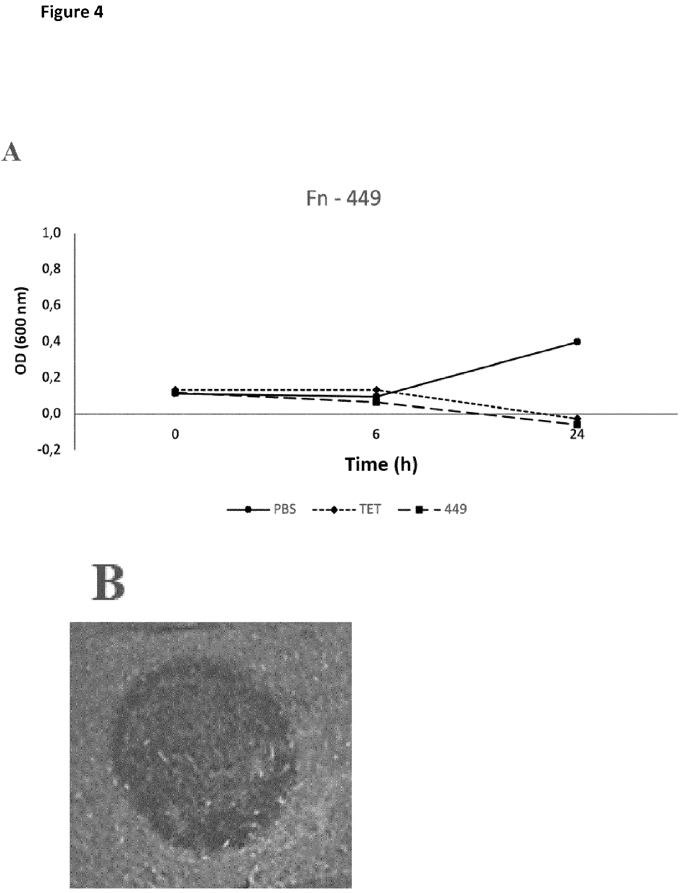

Peptides 125, 353, 355, 410, 413, 426, 434 and 449 form the set of eight selected peptides. Figure 1 shows the activity of peptide 434 on *F. nucleatum,* which at a single dose of 50 µM inhibited the planktonic growth of this bacteria for at least 24 hours. On the other hand, peptides 353, 355 and 449 inhibited the planktonic growth of *F. nucleatum* (Figures 2A, 3A and 4A) and, like 434, the inhibition was maintained for at least 24 hours. The three peptides also inhibited the growth of *F. alocis* (Figures 2B, 3B and 4B); however, peptide 355 showed a higher level of inhibition for this bacterium.

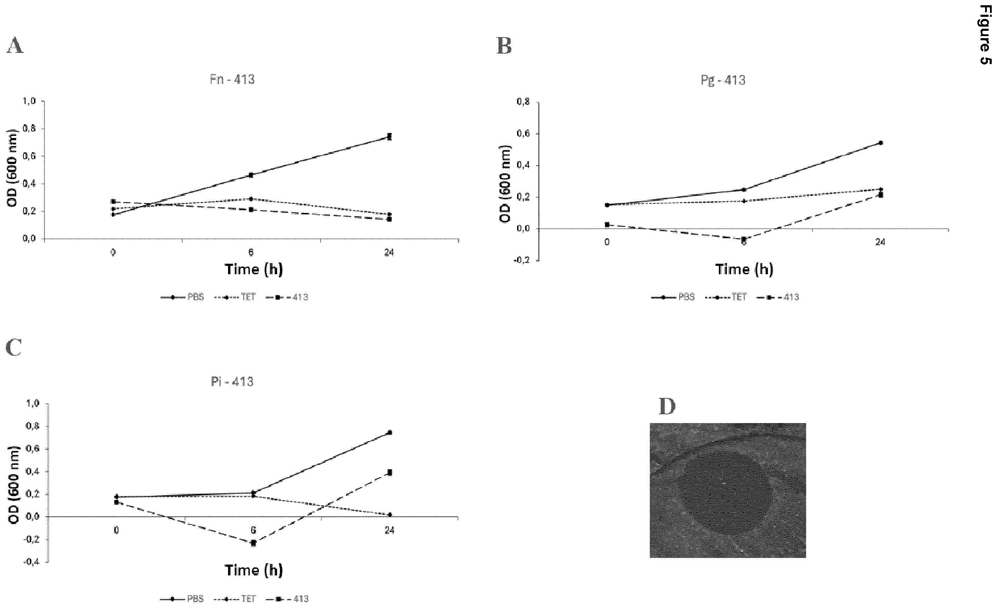

Peptide 413 inhibited the planktonic growth of *F. nucleatum, P. gingivalis* and *P*. *intermedia* and, in the drop test, of *F. alocis* (Figure 5). A single dose of the peptide maintained the inhibition of *P*. *gingivalis* and *P*. *intermedia* for 6 hours (5B and 5C); however *F. nucleatum* growth was inhibited for at least 24 hours (5A).

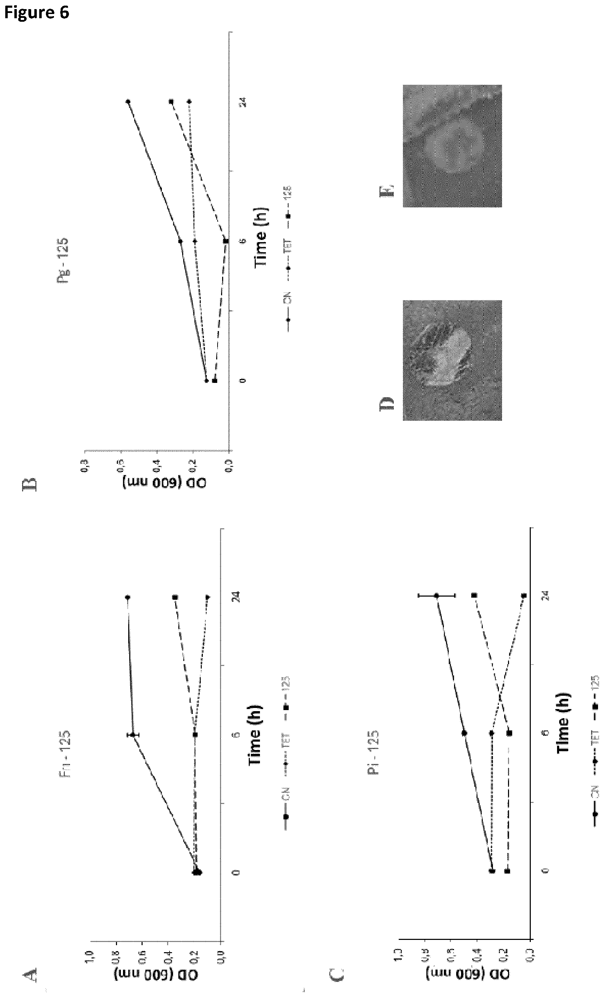

Figure 6 shows that peptide 125 inhibited the growth of the species *F. nucleatum, P. gingivalis, P. intermedia, F. alocis* and *P. endodontalis.* In planktonic culture, the inhibition was maintained for up to 6 hours, after which the 3 species began to grow. However, after 24 hours, their growth did not reach the level of the control without the peptide. (6A, 6B and 6C).

Peptide 426 inhibited the planktonic growth of *A. actinomycetemcomitans, F. nucleatum, P. gingivalis* and *P. intermedia.* In the drop test, this peptide inhibited the growth of *F. alocis.* In the first two species, the inhibition of growth was maintained for at least 24 hours (Figures 6A and 6B). However, for the species *P*. *gingivalis* and *P. intermedia*, growth was inhibited for up to 6 hours; after 24 hours, the growth of both species practically reached the level of the control curve (6C and 6D).

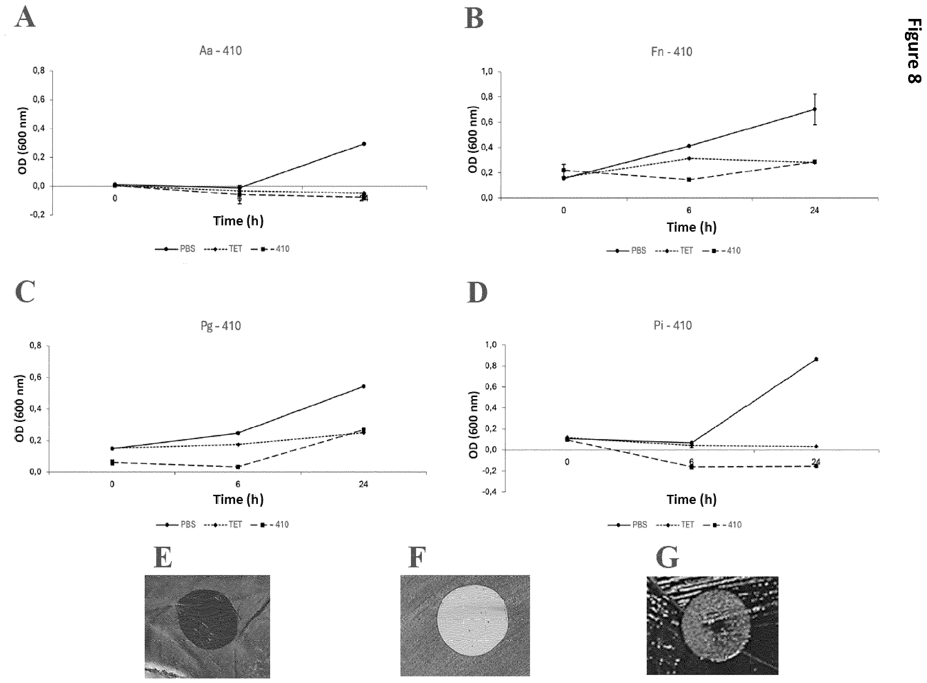

Figure 8 shows the effect of peptide 410 on the 7 pathogenic species used in this study. In planktonic cultures, the behaviour of this peptide was very similar to that of Tetracycline (positive control) and in *A. actinomycetemcomitans, F. nucleatum, P. gingivalis* and *P*. *intermedia,* practically no growth was observed until 24 hours (Figure 8 A-D). Furthermore, the drop test showed that peptide 410 inhibited the growth of *T. forsythia, F. alocis* and *P*. *endodontalis* (Figure 8 E-G).

### Structural Characterisation of the Peptides

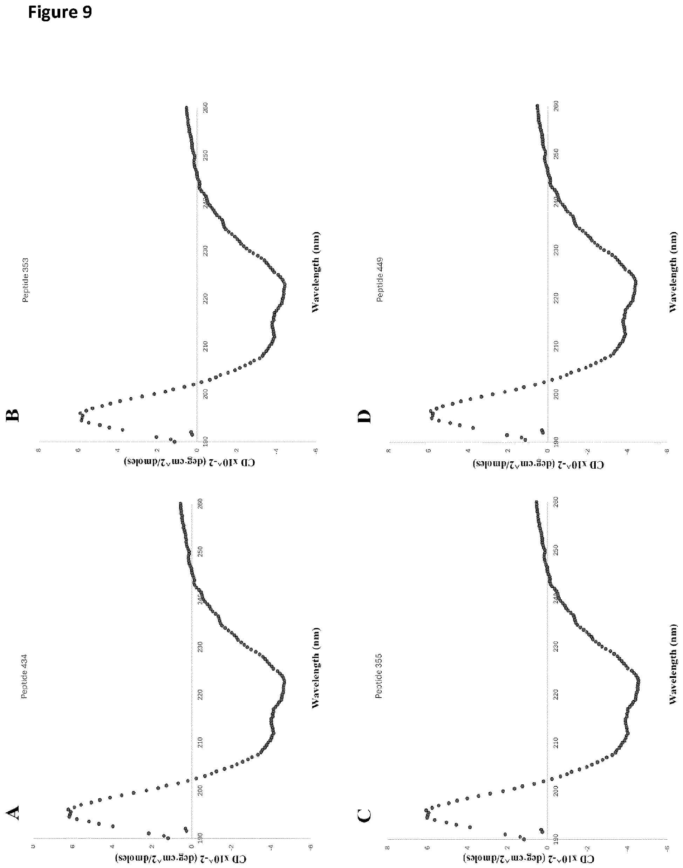

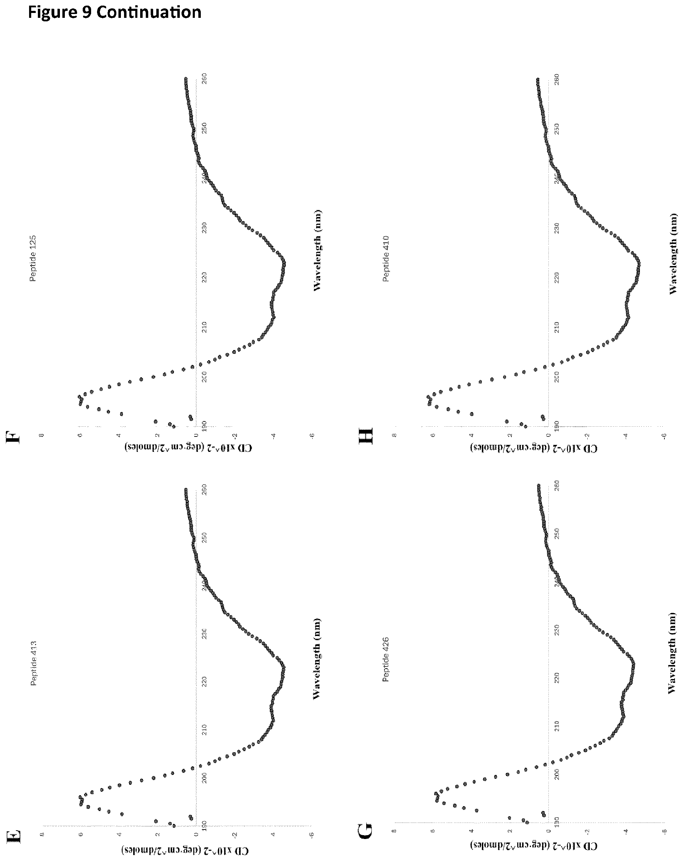

Circular dichroism (CD) analysis of the peptides allowed estimating the type and proportion of the different canonical secondary structures (α-helix, parallel β-sheet, random coil, etc.) present in each peptide in solution. The CD data showed that the studied peptides mostly adopt an α-helix type secondary structure in solution, both in water and in PBS pH 7.4 (Figure 9 A-H). These data are consistent with the molecular modelling used to create the peptide library from which the eight analysed peptides are derived. In addition, through the use of turbidimetry it can be stated that the eight studied peptides do not present a tendency to form fibres or aggregates under the test conditions.

### Peptide Stability

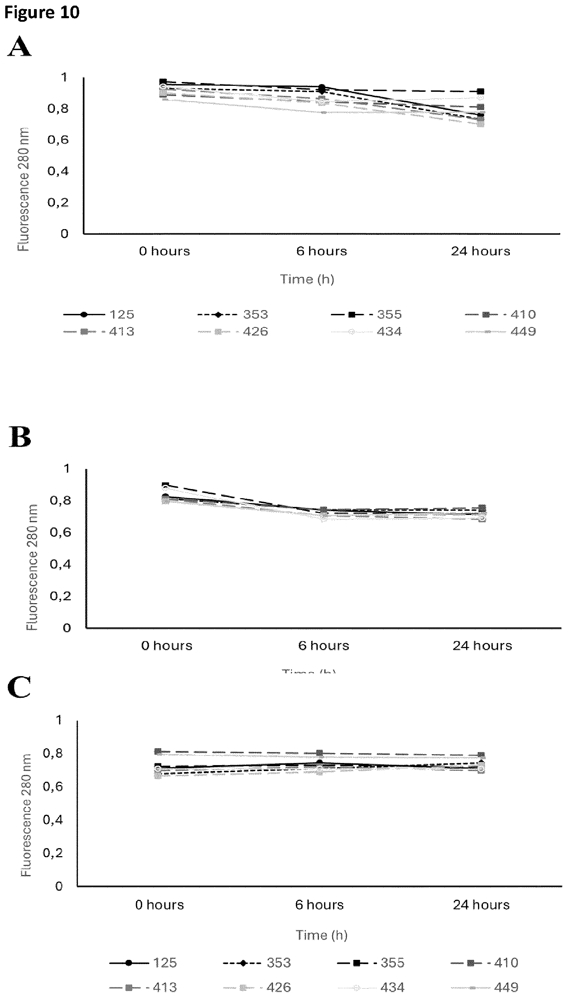

The stability of the peptides as a function of ionic strength was studied by incubating each peptide at 37°C with increasing dilutions of NaCl ranging from 0.01 M to 1.0 M, for up to 72 hours. The results of the fluorescence measured at 280 nm (intrinsic to the aromatic residues) confirm that the peptides are stable in this NaCl concentration range (Figure 10 A-C).

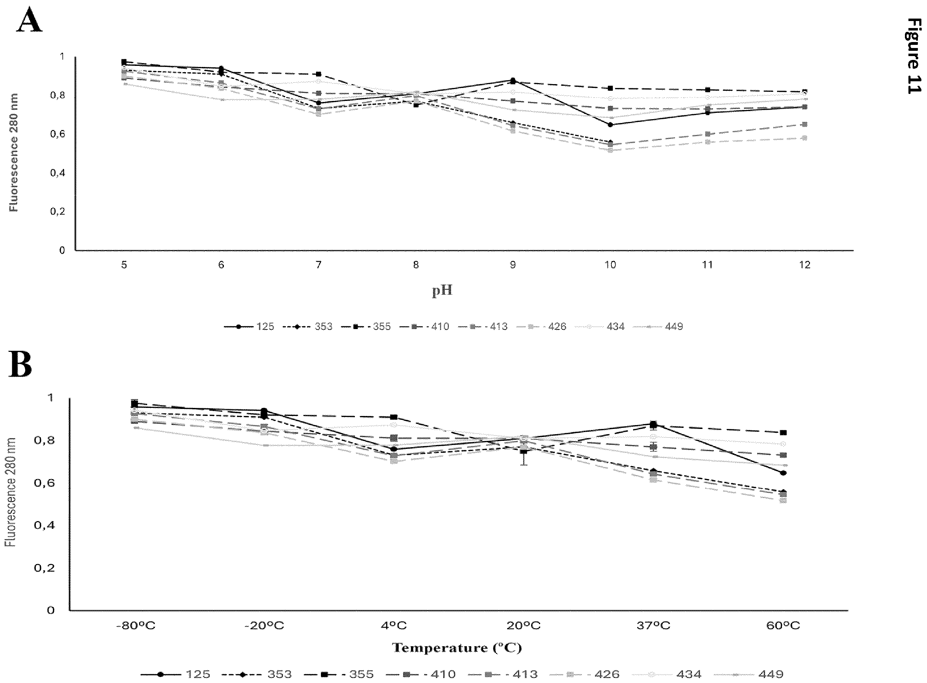

The influence of pH was determined by incubating the peptides for up to 72 hours in solutions with a pH range of 5 to12. The graph in Figure 11A shows that the peptides are stable, particularly in the pH range of 5.5 to 7.5, which is typical for salivary pH. As can be seen in the graph in Figure 11B, the peptides are stable over a wide temperature range. Moreover, the antimicrobial activity tests after incubating the peptides for 72 hours at 37°C were similar to the initial experiments.

### Effect of Peptides on Commensal Bacteria

The activity of the peptides on commensal bacteria was tested using the drop test (Figure 12). Most of the peptides show either no activity or very minimal activity against most species. Peptide 355 exhibits no activity against any of the commensal species, while peptides 353 and 449 show very minimal activity against *S*. *salivarius* and peptide 434 shows very minimal activity against *S*. *oralis.* The peptide with the widest range of activity was 410. Although the activity is minimal against most species, peptide 410 exhibited high activity against *A. naeslundii.* Furthermore, Table 1 shows that the most sensitive species are *V. disparand V. parvula,* which are slightly sensitive to peptides 125, 410, 413 and 426; however, the least sensitive species were *R. mucilaginosa,* which is slightly sensitive to peptide 410, and *N. subflava,* which is not sensitive to any of the eight peptides.

**Table 1**

| | Peptide activity | | | | | | | |
|---|---|---|---|---|---|---|---|---|
| | 125 | 353 | 355 | 410 | 413 | 426 | 434 | 449 |
| **Pathogenic Bacteria** | | | | | | | | |
| *A. actinomycetemcomitans* | --- | --- | --- | +++ | --- | +++ | --- | --- |
| *F. alocis* | ++ | + | +++ | +++ | +++ | ++ | --- | ++ |
| *F. nucleatum* | +++ | +++ | +++ | +++ | +++ | +++ | +++ | +++ |
| *P. endodontalis* | +++ | --- | --- | + | --- | --- | --- | --- |
| *P. gingivalis* | +++ | --- | --- | +++ | +++ | +++ | --- | --- |
| *P. intermedia* | +++ | --- | --- | +++ | +++ | +++ | --- | --- |
| *T. forsythia* | --- | --- | --- | +++ | --- | --- | --- | --- |

| **Commensal Bacteria** | | | | | | | | |
|---|---|---|---|---|---|---|---|---|
| *A. naeslundii* | + | --- | --- | +++ | --- | ++ | --- | --- |
| *A. viscosus* | --- | --- | --- | + | --- | --- | --- | --- |
| *N. subflava* | --- | --- | --- | --- | --- | --- | --- | --- |
| *R. mucilaginosa* | --- | --- | --- | + | --- | --- | --- | --- |
| *S. mitis* | + | --- | --- | + | --- | --- | --- | --- |
| *S. oralis* | + | --- | --- | + | --- | --- | + | --- |
| *S. salivarius* | --- | + | --- | + | + | --- | --- | + |
| *V. dispar* | ++ | --- | --- | ++ | + | ++ | --- | --- |
| *V. parvula* | + | --- | --- | ++ | --- | ++ | --- | --- |

Other peptides belonging to the library of 645 peptides and out of the 8 selected peptides did not show antimicrobial activity against *E. coli*, even though CD determined that they tended to adopt an α-helix structure, for example, peptides 2 (EDQRRDCRREDYCEWARE-SEQ ID NO: 10) and 111 (IYDKKECRKNDDPIEARD-SEQ ID NO: 11). Other peptides, on the other hand, that did show activity against *E. coli* did not show activity against oral bacteria, for example peptide 96 (KMLKKARRRIECLFYDRE-SEQ ID NO: 12).

### REFERENCES

1.- Chen, M. X., Zhong, Y. J., Dong, Q. Q., Wong, H. M., & Wen, Y. F. (2021). Global, regional, and national burden of severe periodontitis, 1990-2019: An analysis of the Global Burden of Disease Study 2019. Journal of clinical periodontology, 48(9), 1165-1188. https://doi. org/10.1111/jcpe.13506
2.- Hajishengallis, G., & Chavakis, T. (2021). Local and systemic mechanisms linking periodontal disease and inflammatory comorbidities. Nature reviews. Immunology, 21(7), 426-440. https://doi.org/10.1038/s41577-020-00488-6
3.- Thomas, C., Minty, M., Vinel, A., Canceill, T., Loubières, P., Burcelin, R., Kaddech, M., Blasco-Baque, V., & Laurencin-Dalicieux, S. (2021). Oral Microbiota: A Major Player in the Diagnosis of Systemic Diseases. Diagnostics (Basel, Switzerland), 11(8), 1376. https://doi.orq/10.3390/diagnostics11081376.
4.- Kwon, T., Lamster, I. B., & Levin, L. (2021). Current Concepts in the Management of Periodontitis. International dental journal, 71(6), 462-476. https://doi.org/10.1111/idj.12630.
5.- Jakubovics, N. S., Goodman, S. D., Mashburn-Warren, L., Stafford, G. P., & Cieplik, F. (2021). The dental plaque biofilm matrix. Periodontology 2000, 86(1), 32-56. https://doi.org/10.1111/prd.12361.
6.- Chapple, I. L., Van der Weijden, F., Doerfer, C., Herrera, D., Shapira, L., Polak, D., Madianos, P., Louropoulou, A., Machtei, E., Donos, N., Greenwell, H., Van Winkelhoff, A. J., Eren Kuru, B., Arweiler, N., Teughels, W., Aimetti, M., Molina, A., Montero, E., & Graziani, F. (2015). Primary prevention of periodontitis: managing gingivitis. Journal of clinical periodontology, 42 Suppl 16, S71-S76. https://doi.org/10.1111/jcpe. 12366.
7.- Figuero, E., Herrera, D., Tobias, A., Serrano, J., Roldán, S., Escribano, M., & Martin, C. (2019). Efficacy of adjunctive anti-plaque chemical agents in managing gingivitis: A systematic review and network meta-analyses. Journal of clinical periodontology, 46(7), 723-739. https://doi.org/10.1111/jcpe.13127.
8.- Pignatelli, P., Fabietti, G., Ricci, A., Piattelli, A., & Curia, M. C. (2020). How Periodontal Disease and Presence of Nitric Oxide Reducing Oral Bacteria Can Affect Blood Pressure. International journal of molecular sciences, 21(20), 7538. https://doi.org/10.3390/ijms21207538.
9.- Tribble, G. D., Angelov, N., Weltman, R., Wang, B. Y., Eswaran, S. V., Gay, I. C., Parthasarathy, K., Dao, D. V., Richardson, K. N., Ismail, N. M., Sharina, I. G., Hyde, E. R., Ajami, N. J., Petrosino, J. F., & Bryan, N. S. (2019). Frequency of Tongue Cleaning Impacts the Human Tongue Microbiome Composition and Enterosalivary Circulation of Nitrate. Frontiers in cellular and infection microbiology, 9, 39. https://doi.org/10.3389/fcimb.2019.00039.
10.- Xu, L., Shao, C., Li, G., Shan, A., Chou, S., Wang, J., Ma, Q., & Dong, N. (2020). Conversion of Broad-Spectrum Antimicrobial Peptides into Species-Specific Antimicrobials Capable of Precisely Targeting Pathogenic Bacteria. Scientific reports, 10(1), 944. https://doi.org/10.1038/s41598-020-58014-6.
11.- Hirt, H., Hall, J. W., Larson, E., & Gorr, S. U. (2018). A D-enantiomer of the antimicrobial peptide GL13K evades antimicrobial resistance in the Gram positive bacteria Enterococcus faecalis and Streptococcus gordonii. PloS one, 13(3), e0194900. https://doi.org/10.1371/journal.pone.0194900.
12.- Nicolas, I., Bordeau, V., Bondon, A., Baudy-Floc'h, M., & Felden, B. (2019). Novel antibiotics effective against gram-positive and -negative multi-resistant bacteria with limited resistance. PLoS biology, 17(7), e3000337. https://doi.org/10.1371/journal.pbio.3000337.
13.- Zhang, Q. Y., Yan, Z. B., Meng, Y. M., Hong, X. Y., Shao, G., Ma, J. J., Cheng, X. R., Liu, J., Kang, J., & Fu, C. Y. (2021). Antimicrobial peptides: mechanism of action, activity and clinical potential. Military Medical Research, 8(1), 48. https://doi.org/10.1186/s40779-021-00343-2.
14.- Lai, Y., & Gallo, R. L. (2009). AMPed up immunity: how antimicrobial peptides have multiple roles in immune defense. Trends in immunology, 30(3), 131-141. https://doi.org/10.1016/j.it.2008.12.003.
15.- Yang, B., Pang, X., Li, Z., Chen, Z., & Wang, Y. (2021). Immunomodulation in the Treatment of Periodontitis: Progress and Perspectives. Frontiers in immunology, 12, 781378. https://doi.org/10.3389/fimmu.2021.781378.

## Claims

1. A peptide comprising o consisting of the following amino acid structure:
K R W K W X₁ K Y X₂ H R Y F I X₃ H R R (SEQ ID NO: 1)
wherein X₁ is selected from M, K, G, L, **F,** I and T;
wherein X₂ is selected from **F,** W, I, Y, L and V;
and wherein X₃ is selected from M, V, S, K, L, I and Q.

2. A peptide cocktail comprising or consisting of at least two of the peptides according to claim 1.

3. A peptide cocktail comprising or consisting of at least three of the peptides according to claim 1.

4. The peptide cocktail according to claim 3, wherein a first peptide cocktail comprises or consists of the following peptides:
a1) K R W K W K K Y I H R Y F I V H R R (SEQ ID NO: 2)
a2) K R W K W G K Y W H R Y F I V H R R (SEQ ID NO: 3)
a3) K R W K W T K Y V H R Y F I Q H R R (SEQ ID NO: 4).

5. The peptide cocktail according to claim 1, wherein a second peptide cocktail comprises the following peptides:
b1) K R W K W M K Y F H R Y F I M H R R (SEQ ID NO: 5)
b2) K R W K W F K Y Y H R Y F I K H R R (SEQ ID NO: 6)
b3) K R W K W I K Y L H R Y F I L H R R (SEQ ID NO: 7).

6. The peptide cocktail according to claim 5, wherein a second peptide cocktail consists of the peptides:
b1) K R W K W M K Y F H R Y F I M H R R (SEQ ID NO: 5)
b2) K R W K W F K Y Y H R Y F I K H R R (SEQ ID NO: 6)
b3) K R W K W I K Y L H R Y F I L H R R (SEQ ID NO: 7).

7. The peptide cocktail according to claim 1, wherein a third peptide cocktail comprises the following peptides:
c1) K R W K W M K Y F H R Y F I M H R R (SEQ ID NO: 5)
c2) K R W K W L K Y I H R Y F I S H R R (SEQ ID NO: 8)
c3) K R W K W F K Y Y H R Y F I K H R R (SEQ ID NO: 6).

8. The peptide cocktail according to claim 7, wherein a third peptide cocktail consists of the peptides:
c1) K R W K W M K Y F H R Y F I M H R R (SEQ ID NO: 5)
c2) K R W K W L K Y I H R Y F I S H R R (SEQ ID NO: 8)
c3) K R W K W F K Y Y H R Y F I K H R R (SEQ ID NO: 6).

9. The peptide cocktail according to claim 1, wherein a fourth peptide cocktail comprises the following peptides:
d1) K R W K W M K Y F H R Y F I M H R R (SEQ ID NO: 5)
d2) K R W K W L K Y I H R Y F I S H R R (SEQ ID NO: 8)
d3) K R W K W I K Y Y H R Y F I I H R R (SEQ ID NO: 9).

10. The peptide cocktail according to claim 9, wherein a fourth peptide cocktail consists of the peptides:
d1) K R W K W M K Y F H R Y F I M H R R (SEQ ID NO: 5)
d2) K R W K W L K Y I H R Y F I S H R R (SEQ ID NO: 8)
d3) K R W K W I K Y Y H R Y F I I H R R (SEQ ID NO: 9).

11. A pharmaceutical composition comprising the peptide or peptide cocktail according to any of claims 1 to 10.

12. The peptide or peptide cocktail according to any of claims 1 to 10 or composition according to claim 11 for use in the prevention and/or treatment of an oral inflammatory disease induced by oral pathogenic bacteria or any systemic disease which is negatively affected by said pathogenic bacteria.

13. The peptide or peptide cocktail or composition for use according to claim 12, wherein said oral pathogenic bacteria are selected from *Porphyromonas gingivalies, Tannerella forsythia, Filifactor alocis, Fusobacterium nucleatum, Aggregatibacter actinomycetemcomitans, Prevotella intermedia and Porphyromonas* endodontalis.

14. The peptide or peptide cocktail or composition for use according to claim 12 or 13, wherein said oral inflammatory disease is selected from gingivitis, periodontitis, mucositis and peri-implantitis.

15. The peptide or peptide cocktail or composition for use according to any of claims 12 to 14, wherein said peptide cocktail or composition is used or administered in the form of a gel, toothpaste, lyophilized powders, chewing gum or chewable product.
